**Europäisches Patentamt**

**European Patent Office**

**Office européen des brevets**

(19)

(11) Veröffentlichungsnummer: **0 008 369**
**B2**

(12) # NEUE EUROPÄISCHE PATENTSCHRIFT

(45) Veröffentlichungstag der neuen Patentschrift:
06.06.84

(51) Int. Cl.³: **C 07 C 102/00**, C 07 C 103/90

(21) Anmeldenummer: **79102533.1**

(22) Anmeldetag: **18.07.79**

(54) **Verfahren zur Herstellung von Tetraacetyläthylendiamin.**

(30) Priorität: **21.07.78 DE 2832021**

(43) Veröffentlichungstag der Anmeldung:
**05.03.80 Patentblatt 80/5**

(45) Bekanntmachung des Hinweises auf die Patenterteilung:
**15.07.81 Patentblatt 81/28**

(45) Bekanntmachung des Hinweises auf die Entscheidung
über den Einspruch:
**06.06.84 Patentblatt 84/23**

(84) Benannte Vertragsstaaten:
**BE DE FR GB IT NL**

(56) Entgegenhaltungen:
**DE - C - 2 118 281**
**DE - C - 2 118 282**

**R.P. Mariella and K.H. Brown: "Diacetylation of Amines"**
**abgedruckt in J. Org. Chem., Vol. 36, No. 5, 1971, S.**
**735-737**
**Ph. D. Dissertation of K.H. Brown, Loyola University**
**1970, S. 28, 85+89**

(73) Patentinhaber: **BASF Aktiengesellschaft,**
**Carl-Bosch-Strasse 38, D-6700 Ludwigshafen (DE)**

(72) Erfinder: **Bott, Kaspar, Dr. Chem., Rieslingweg 4,**
**D-6706 Wachenheim (DE)**
Erfinder: **Hoffmann, Herwig, Dr. Chem.,**
**Knietschstrasse 21, D-6710 Frankenthal (DE)**
Erfinder: **Wulz, Klaus, Dr. Chem., Ungsteiner Strasse 22,**
**D-6700 Ludwigshafen (DE)**
Erfinder: **Wostbrock, Karl-Heinz, Dr. Chem.,**
**Amsterdamer Strasse 16, D-6700 Ludwigshafen (DE)**

## Beschreibung

Gegenstand der Erfindung ist ein Verfahren zur kontinuierlichen Herstellung von Tetraacetyläthylendiamin durch unmittelbare, d. h. einstufige Reaktion von Acetanhydrid mit Äthylendiamin in einer Destillationskolonne mit entsprechendem Rückhaltevermögen, beispielsweise einer Glockenbodenkolonne. Es besteht darin, daß man Äthylendiamin mit stöchiometrischer Menge oder mit überschüssigem Acetanhydrid bei einer Temperatur von 120 bis 190° C einstufig in einer Destillationskolonne umsetzt, wobei man das Wasser und die Essigsäure in dem Maße, wie sie entstehen abdestilliert, das ggf. Acetanhydrid enthaltende Tetraacetyläthylendiamin am Fuß der Kolonne gewinnt und ggf. nach Abtrennung und Rückführung von Acetanhydrid unter vermindertem Druck destilliert.

Tetraacetyläthylendiamin wirkt als Kaltbleichaktivator für perborathaltige Waschmittel: Es ermöglicht eine Herabsetzung der Waschtemperatur, die für die Reinigung von Textilien aus Synthesefasern und von Buntwäsche besonders erwünscht ist.

Aus den deutschen Patentschriften 2 118 281 und 2 118 282 ist die bisher beste Methode zur Gewinnung von Tetraacetyläthylendiamin in technischem Maßstab bekannt, die in der stufenweisen Umsetzung von Äthylendiamin mit zunächst Essigsäure und dann Acetanhydrid über die Zwischenstufe des N,N'-Di-acetyläthylendiamins in einer Kaskade von mehreren Rührkesseln besteht. Das N,N'-Diacetyläthylendiamin muß somit bei technischer Verfahrensgestaltung nach bisheriger Erkenntnis als solches gewonnen und dann weiter umgesetzt werden. Dieser Prozeß hat den Nachteil — abgesehen von dem offensichtlichen Umstand der Mehrstufigkeit —, daß trotz des hohen Acetanhydrid-Bedarfs kein vollständiger Umsatz erreicht wird und daß man deshalb eine aufwendige Trennung und Stoffrückführung betreiben muß. Ein weiterer Nachteil dieses Verfahren besteht darin, daß das Rohprodukt durch Auskristallisation aus Acetanhydrid gereinigt werden muß und somit einen zusätzlichen Materialkreislauf erfordert.

Zwar ist aus der Dissertation von K. H. Brown, Loyola Universität, Chicago 1970, S. 28, 85 und 89 bzw. einer Veröffentlichung in J. Org. Chem. 36, 735 bis 737 bekannt, N,N, N',N'-Tetraacetylethylendiamin auch einstufig, d. h. unmittelbar ausgehend von Ethylendiamin und Acetanhydrid herzustellen. Bei diesem, absatzweise und im Labormaßstab ausgeführten Verfahren wurden jedoch bei 20stündiger Reaktion nur 25% Ausbeute erreicht, obwohl offensichtlich — da kein Diacetylethylendiamin gefunden wurde — ein erheblicher Überschuß an Acetanhydrid angewendet worden sein muß.

Die Aufgabe, ein unmittelbar von Ethylendiamin zum Zielprodukt führendes technisches Verfahren zu schaffen, mußte daher noch als ungelöst gelten, zumal wenn dabei mindestens ein Ausgangsstoff vollständig umgesetzt und möglichst wenig Acetanhydrid gebraucht werden sollte.

Es wurde gefunden, daß sich Tetraacetyläthylendiamin günstiger in einem Verfahrensschritt gewinnen läßt, wobei man Äthylendiamin unmittelbar mit einer stöchiometrischen Menge oder mit überschüssigem Acetanhydrid bei 120 bis 190, vorzugsweise 140 bis 190° C zur Umsetzung bringt. Die Erfindung besteht darin, daß man die Umsetzung kontinuierlich in einer Destillationskolonne vornimmt, wobei man das Wasser und die Essigsäure in dem Maße, wie sie entstehen abdestilliert, das ggf. Acetanhydrid enthaltende Tetraacetyläthylendiamin am Fuß der Kolonne gewinnt und ggf. nach Abtrennung und Rückführung von Acetanhydrid unter vermindertem Druck destilliert.

Es ist zweckmäßig, wenn man das Acetanhydrid — vorzugsweise gasförmig — in den unteren Teil z. B. einer Glockenbodenkolonne bzw. flüssig in einen unten an die Kolonne sich anschließenden Verdampfer einführt und das Äthylendiamin am Kolonnenkopf in flüssiger Form einleitet. Die Kolonne sollte eine Verweilzeit von mindestens einer, besser noch mindestens zwei Stunden erlauben und somit ein hinreichendes Flüssigkeitsrückhaltevermögen (hold up) besitzen. Es versteht sich, daß auch eine ausreichende Trennleistung vorhanden sein muß. Zweckmäßig soll die Kolonne nicht weniger als 5, vorzugsweise mindestens 10 theoretische Böden aufweisen. Im allgemeinen sind Verweilzeiten von mehr als 10 Stunden und Trennleistungen von mehr als 30 theoretischen Böden nicht erforderlich.

Das am Fuß der Kolonne anfallende Reaktionsprodukt wird am besten unmittelbar in eine Verdampfungsvorrichtung — z. B. den vorerwähnten Verdampfer — eingeleitet, um das überschüssige Acetanhydrid aus dem Reaktionsprodukt zu entfernen und sogleich in den Reaktor zurückzuführen. Am Kolonnenkopf destilliert — je nach Molverhältnis der Ausgangsstoffe — ein Gemisch von Wasser und Essigsäure oder Essigsäure ab. Das Molverhältnis beträgt vorzugsweise 1 : 2,1 bis 1 : 10, insbesondere 1 : 2,5 bis 4. Die Anwendung stöchiometrischer oder geringerer Mengen Acetanhydrid führt zu Produkten geringerer Reinheit. Um den Transport der gebildeten Essigsäure in den oberen Kolonnenteil zu verstärken, kann man z. B. einen schwachen Stickstoffstrom durch die Kolonne leiten. Auch kann entlang der Kolonne eine unterschiedliche Temperatur, zweckmäßig von unten nach oben zunehmend, eingestellt werden. Im oberen Teil wird zweckmäßig eine Temperatur von 150 bis 190° C eingehalten.

Das anfallende Reaktionsgemisch besitzt nach dem Abdestillieren von Acetanhydrid schon einen Gehalt von 93 bis 96% an aktiven Acetylgruppen, berechnet auf reines Tetraacetyläthylendiamin. Die Bestimmung des Acetylgruppengehalts kann z. B. acidimetrisch vorgenommen werden.

Die Erfindung erlaubt es, das erhaltene rohe Tetraacetyläthylendiamin bei vermindertem Druck, z. B. 1 bis 10 mbar und einer Siedetemperatur von 155 bis 170°C ohne größere Rückstandsbildung (erfahrungsgemäß 4 bis 6% der Gesamtmenge) zu destillieren und somit zu reinigen. Dies verdient deshalb Beachtung, weil bisher schon bei der Synthese des Tetraacetyläthylendiamins unter ähnlichen Temperaturbedingungen schwarze, teerartige Nebenprodukte gebildet wurden, so daß die Destillation unter vergleichbaren Temperaturbedingungen als Reinigungsmethode bisher nicht anwendbar galt.

Die Destillation kann natürlich ebenso absatzweise wie fortlaufend geschehen, vorteilhaft ist die fortlaufende Dünnschichtverdampfung. Das durch Destillation gereinigte Tetraacetyläthylendiamin unterscheidet sich hinsichtlich der Kaltbleichwirkung beim Zusatz zu perborathaltigen Waschmitteln nicht von der durch Umkristallisation gewonnenen Ware.

Beispiel 1

Zur Vorführung des Verfahrens wird eine Glockenbodenkolonne mit 20 Böden mit einem nutzbaren Volumen von 700 ml verwendet. Sie ist mit einem Doppelmantel ausgerüstet, durch den eine Heizflüssigkeit gepumpt werden kann. Zur Entfernung von Acetanhydrid aus dem aus der Kolonne abfließenden Reaktionsprodukt ist unmittelbar unter die Kolonne ein beheizter Dünnschichtverdampfer und hierunter ein Auffanggefäß angeordnet, welches mit einer Stickstoffleitung in Verbindung steht. Die Glockenbodenkolonne besitzt einen mit Füllkörpern versehenen Destillationsaufsatz von 20 cm Länge, der am oberen Ende mit einem Liebig-Kühler verbunden ist. Es wird ein Stickstoffstrom von stündlich 12 l eingestellt. Zur Vorführung der Reaktion fördert man stündlich 103 g (etwa 1 Mol) technisches Acetanhydrid in den oberen Teil des Dünnschichtverdampfers, wo es verdampft und 15 g (etwa 0,25 Mol) technisches Äthylendiamin auf den obersten Glockenboden der Kolonne. Die Temperaturen der Heizflüssigkeiten betragen für die Reaktionskolonne 145°C und für den Dünnschichtverdampfer 175°C. Dabei destillieren stündlich 62 g 99%ige Essigsäure ab, während 56 g rohes Tetraacetyläthylendiamin ausgetragen werden. Durch Anlegen eines Wasserstrahlvakuums wird das Rohprodukt bei 150°C vom restlichen Acetanhydrid (2%, bezogen auf den Gesamtaustrag) befreit. Es besitzt einen acidimetrisch bestimmten Gehalt an aktiven Acetylgruppen von 94%, (berechnet auf reines Tetraacetyläthylendiamin.

Beispiel 2

Für diesen Versuch stand eine Kolonne mit 21 Glockenböden und einem Flüssigkeitsvolumen (hold up) von 1,1 Liter zur Verfügung. Die sieben oberen Glockenböden wurden auf eine Temperatur von 165—170°C geheizt, während die Reaktionstemperatur im übrigen Kolonnenteil bei 150°C gehalten wurde. Der übrige Aufbau entsprach dem vorerwähnten.

Es wurden stündlich 176 g Acetanhydrid und 28 g Äthylendiamin in der vorstehend beschriebenen Weise zugeführt. Außerdem wurden 9 l Stickstoff pro Stunde durch die Apparatur geleitet. Als Destillat wurden 36 g 58%ige Essigsäure erhalten; die Menge des Sumpfprodukts betrug 168 g an, in der noch 67 g Acetanhydrid enthalten waren. Das nach dem Abdestillieren niedrig siedender Begleitstoffe verbleibende rohe Tetraacetyläthylendiamin wies bei der acidimetrischen Bestimmung einen Gehalt von 95% an aktiven Acetylgruppen auf. Unter Berücksichtigung des wiedergewonnenen Acetanhydrids wurden bei diesem Versuch 2,3 Mol Acetanhydrid je 1 Mol Äthylendiamin verbraucht.

Zur Reinigung des Tetraacetyläthylendiamins wurden 586 g Rohprodukt aus einem der vorstehend beschriebenen Versuche in einem Glasapparat mit Claisenaufsatz und Luftkühler bei einem Druck von 2,5 mbar destilliert. Die Hauptmenge des Destillats geht bei 165°C über. Man gewinnt 554 g Tetraacetyläthylendiamin mit einer Farbzahl von 15 Hazen-Graden. Der Destillationsrückstand wiegt 28 g (4,8%, bezogen auf das Rohprodukt).

**Patentansprüche**

1. Verfahren zur Herstellung von N,N,N'N'-Tetraacetyläthylendiamin, wobei man Äthylendiamin mit einer stöchiometrischen Menge oder mit überschüssigem Acetanhydrid bei einer Temperatur von 120 bis 190°C einstufig umsetzt, dadurch gekennzeichnet, daß man die Umsetzung kontinuierlich in einer Destillationskolonne vornimmt, wobei man das Wasser und die Essigsäure in dem Maße, wie sie entstehen abdestilliert, das ggf. Acetanhydrid enthaltende Tetraacetyläthylendiamin am Fuß der Kolonne gewinnt und ggf. nach Abtrennung und Rückführung von Acetanhydrid unter vermindertem Druck destilliert.

2. Verfahren nach Anspruch 1, dadurch gekennzeichnet, daß man unter Berücksichtigung des zurückgeführten Acetanhydrids ein Molverhältnis von Äthylendiamin zu Acetanhydrid von 1 : 2,1 bis 1 : 10 einhält.

3. Verfahren nach Anspruch 1, dadurch gekennzeichnet, daß man als Destillationskolonne eine Kolonne verwendet, die eine Verweilzeit des Reaktionsgemisches von wenigstens einer Stunde bei einer theoretischen Bodenzahl von wenigstens 5 erlaubt.

4. Verfahren nach Anspruch 1, dadurch gekennzeichnet, daß man eine Glockenbodenkolonne verwendet.

5. Verfahren nach Anspruch 1, dadurch gekennzeichnet, daß man im oberen Teil der Kolonne eine höhere Temperatur einhält als im mittleren bzw. unteren Teil.

6. Verfahren nach Anspruch 5, dadurch gekennzeichnet, daß man im oberen Teil der Kolonne eine Temperatur von 150 bis 190° C einhält.

**Claims**

1. A process for the production of N,N,N′,N′-tetraacetylethylenediamine by reacting ethylenediamine with a stoichiometric amount or an excess of acetic anhydride at a temperature of from 120° to 190°C in a single step, characterized in that the reaction is carried out continuously in a distillation column, the water and acetic acid being distilled off as they are formed, the tetraacetylethylenediamine which may contain acetic anhydride is recovered at the bottom of the column and distilled under reduced pressure after separating off and recycling any acetic anhydride present.

2. A process as claimed in claim 1, characterized in that a molar ratio of ethylenediamine to acetic anhydride of from 1 : 2.1 to 1 : 10 is maintained, taking the recycled acetic anhydride into account.

3. A process as claimed in claim 1, characterized in that there is used as distillation column a column which permits the reaction mixture to reside therein for at least one hour, the number of theoretical trays being at least 5.

4. A process as claimed in claim 1, characterized in that a bubble-cap column is used.

5. A process as claimed in claim 1, characterized in that a higher temperature is maintained in the upper section of the column than in the middle and lower sections.

6. A process as claimed in claim 5, characterized in that a temperature of from 150° to 190°C is maintained in the upper section of the column.

**Revendications**

1. Procédé pour la préparation de N,N,N′,N′tétraacétyléthylène-diamine par la mise en réaction en un seul temps d'éthylène-diamine avec une quantité stoechiométrique ou un excès d'anhydride acétique à une température de 120 à 190°C, caractérisé en ce qu'on procède à la réaction de façon continue dans une colonne de distillation, en chassant par distillation l'eau et l'acide acétique dans la mseure où ils se forment, en récupérant au pied de la colonne la tétraacétyléthylène-diamine qui contient éventuellement de l'anhydride acétique et en la distillant sous pression réduite, le cas échéant après séparation et recyclage de l'anhydride acétique.

2. Procédé selon la revendication 1, caractérisé en ce qu'en tenant compte de l'anhydride acétique recyclé, on maintient un rapport molaire de l'éthylène-diamine à l'anhydride acétique de 1 : 2,1 à 1 : 10.

3. Procédé selon la revendication 1, caractérisé en ce qu'on utilise, en tant que colonne de distillation, une colonne qui permet une durée de séjour du mélange réactionnel d'au moins une heure, avec un nombre de plateaux théoriques d'au moins 5.

4. Procédé selon la revendication 1, caractérisé en ce qu'on utilise une colonne à plateaux à calottes.

5. Procédé selon la revendication 1, caractérisé en ce qu'on maintient, dans la partie supérieure de la colonne, une température plus élevée que dans sa partie moyenne ou sa partie inférieure.

6. Procédé selon la revendication 5, caractérisé en ce qu'on maintient, dans la partie supérieure de la colonne, une température de 150 à 190°C.